(19) Europäisches Patentamt / European Patent Office / Office européen des brevets

(11) **EP 4 406 502 A1**

(12) **EUROPEAN PATENT APPLICATION**

(43) Date of publication:
**31.07.2024 Bulletin 2024/31**

(21) Application number: **23153173.2**

(22) Date of filing: **24.01.2023**

(51) International Patent Classification (IPC):
**A61B 34/20** (2016.01)   **A61B 90/00** (2016.01)
**G09B 23/28** (2006.01)

(52) Cooperative Patent Classification (CPC):
**A61B 34/20; A61B 90/36; G09B 23/285;**
A61B 2034/2048; A61B 2034/2051;
A61B 2034/2061; A61B 2034/2065;
A61B 2090/365; A61B 2090/372; A61B 2090/374;
A61B 2090/376

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicants:
• **Barco N.V.**
  **8500 Kortrijk (BE)**
• **Katholieke Universiteit Leuven, KU Leuven R&D**
  **3000 Leuven (BE)**

(72) Inventors:
• **VÖRÖS, Viktor**
  **3010 Kessel-Lo (BE)**
• **VANDER POORTEN, Emmanuel**
  **2800 Mechelen (BE)**

(74) Representative: **IPLodge bv**
  **Technologielaan 9**
  **3001 Heverlee (BE)**

(54) **SYSTEM AND METHOD FOR VISUAL IMAGE GUIDANCE DURING A MEDICAL PROCEDURE**

(57)   The present invention relates to a system for visualization during a medical procedure, the system comprising:
• imaging data;
• tracking data of one or more instruments that are inserted in a patient's body or a patient model;
• pose data of the patient's body or the patient model;
• processing means for co-registering the imaging data, tracking data and pose data;
• a display for simultaneous visualization of the co-registered data;

the system being characterized in that
• the tracking data comprises three or more non-colinear tracking points, and/or one or more tracking point and an appropriate angle representation, and/or a point cloud representation, for at least one instrument;
• the tracking data is used to determine a position and/or shape and/or orientation of the instrument; and
• the visualization comprises a representation of the shape and/or the position and/or the orientation of the instrument.

Figure 1

## Description

## Field of the invention

The present invention relates to the field of visual image guidance during medical procedures or simulated medical procedures.

## Background

Advancements in medical technologies have enabled the development and regular use of minimally invasive procedures, such as for instance laparoscopy or coronary catheterization. These minimally invasive procedures reduce operative trauma, wound healing time, associated pain and risk of infection and other complications. However, it remains a challenge to provide proper visual guidance to the physician executing these procedures. Typically, physicians rely on intraoperative endoscopy and/or fluoroscopy images, potentially combined with pre-operative imaging techniques such as CT-scan or MRI, as well as their experience and knowledge of human anatomy. This way of working is not without its drawbacks: endoscopes have a limited field of view, fluoroscopy and pre-operative imaging techniques require expensive equipment and expose the patient to harmful radiation. In addition the overwhelming majority of these techniques provide 2D images. The burden of properly estimating the position and orientation, often referred to as pose, of a 3D instrument within the cavity of the patient's body thus still rests on the physician.

Document US20200188028A1 discloses a system and method for providing an augmented reality visualization during a medical procedure on a patient. According to US20200188028A1, a three-dimensional (3D) model of an anatomic part of the patient is presented on a 3D display in alignment with two-dimensional (2D) live imaging data obtained during the procedure. In addition, some embodiments of US20200188028A1 utilize 3D tracking data to localize instruments used in the procedure in real time. The visualization of the position of the instruments can be presented on the 3D display in alignment with the 3D model and/or the 2D live imaging data to guide a clinician in performing the medical procedure.

This solution for visual image guidance during a medical procedure does not consider that the instrument can extend in three dimensions; it tracks one point of the physical instrument and assumes that the corresponding part of the instrument always fits inside the patient's cavity. However, the tip of an endoscope for example can be bent in order to obtain a different camera view angle. If the clinician would rotate such a bent tool around its own axis, inside a patient cavity, he/she could harm the patient's internal and/or wall tissue.

Therefore, there is still a need for an alternative and improved system and method for visual image guidance during a medical procedure.

## Summary

The present invention aims to remedy the above-mentioned and other disadvantages.

According to a first aspect of the invention, a system for providing a visualization during a medical procedure on a patient or a simulated medical procedure on a patient model is disclosed, the system comprising:

a. means for receiving imaging data of a first region of the patient's body or the patient model;
b. means for receiving tracking data of one or more instruments that are inserted in a second region of the patient's body or the patient model;
c. means for receiving pose data of the patient's body or the patient model, the pose data belonging to a third region;
d. processing means for co-registering the imaging data, tracking data and pose data;
e. a display for simultaneous visualization of two or more of the first, second and third regions, and of the co-registered data belonging to the simultaneously visualized regions;
the system being characterized in that
f. the tracking data comprises three or more tracking points, wherein the three tracking points are not colinear; and/or one or more tracking points and a roll, pitch and yaw angle or a set of Euler angles or a set of quaternions or an angle-axis representation or a rotation matrix or a homogeneous transformation matrix; and/or a point cloud representation, for at least one of the one or more instruments;
g. the tracking data is used to determine a position and/or shape and/or orientation of at least one of the one or more instruments; and
h. the visualization comprises a representation of the shape and/or the position and/or the orientation of at least one of the one or more instruments.

In the context of the present invention, "imaging data" refers to data that represents one or more visual images of one or more parts of the interior of the patient's body or the patient model. Alternatively, or in addition, imaging data may represent visual images of one or more parts of the instruments or of a physician operating the instruments. For instance, the imaging data may comprise a visual image of an interior region of the patient's body, together with a part of an instrument inserted into said region and with the physician's hand holding the instrument. Alternatively, or in addition, imaging data may refer to non-visual data of the patient's body or patient model that is recorded, such as for instance heart rate, blood pressure or oxygen saturation levels.

In the context of the present invention, "tracking data" refers to data that can be used to retrieve a spatial position and/or orientation of the one or more instruments. Alternatively, or in addition, tracking data may also comprise time-related data such as time stamps, an

3      **EP 4 406 502 A1**      4

indication of chronology, propagation speeds etcetera.

In the context of the present invention, "pose data" refers to the ensemble of position and orientation. In case of a human patient, the pose data refers amongst other to the position and orientation of the head, trunk and limbs. The pose data may concern the entirety of the patient's body or only a part of it. In case of an instrument, the pose data can refer amongst others to the position and the orientation of a point of the instrument, e.g. the instrument tip, or the instrument handle, or any other convenient and representative point on the instrument.

In the context of the present invention, "co-registration" refers to the process of aligning data that originates in different reference frames. This co-registration may require translating, rotating or scaling data, both in the spatial and the temporal domain.

The tracking data of at least one of the one or more instruments comprises three or more tracking points, wherein the three tracking points are not colinear. Knowledge of the position of three non-colinear tracking points allows to determine the position and orientation of the instrument in an unambiguous manner, when the tracking points have a fixed geometric relationship to the instrument. Preferably, the instrument comprises tracking points that are distributed over its geometrical structure such that the shape of the instrument can be estimated through knowledge of the tracking points.

Alternatively, the tracking data of at least one of the one or more instruments comprises one or more tracking points, and/or representation of the corresponding orientation(s) either by means of the corresponding roll, pitch and yaw angle, set of Euler angles, set of quaternions, angle-axis representation, rotation matrix, homogeneous transformation matrix or any other representation of orientation. Knowledge of these parameters also allows for an unambiguous determination of the position and orientation of the instrument, but only when the tracking point has a fixed or known geometric relationship to the instrument. For instruments that are deformable and adjustable in shape, the shape of the instrument can be estimated provided there are sufficient tracking points distributed over the instrument, or a point cloud representation of the instrument's shape, or a mechanical or other type of relation that allows estimating the entire instrument's shape based on input parameters such as actuation force, pressure or other input parameters.

It is an advantage of the system that not only the shape, but also the position and orientation of the instruments can be visualized. If the shape, position and/or orientation of the instruments are visualized on the display, the physician performing the procedure does not need to rely solely on knowledge, experience and feel to estimate whether the instruments are properly positioned and fit inside the anatomical structure where they are inserted. This may allow for faster, safer and more effective medical procedures.

In some embodiments of the system, the system further comprises means for receiving model data of a fourth region of the patient's body or the patient model. In addition, the processing means are adapted for co-registering the imaging data, tracking data, pose data and model data, and the display is adapted for simultaneous visualization of two or more of the first, second, third and fourth regions, and of the co-registered data belonging to the simultaneously visualized regions.

In the context of the present invention, "model data" refers to data that offers a visual representation of one or more parts of the interior of the patient's body or the patient model. When compared to "imaging data", model data may offer higher spatial resolution. Model data is often based on various sources of imaging data that have been processed by a computer to generate a single, more complex, data set. In addition, model data may comprise visual indications of properties that are not visually discernible in real life.

In some embodiments of the system comprising model data, the model data and tracking data comprise 3D data, the display is a 3D display and a representation of the model data and/or the representation of the shape and/or position and/or orientation of the one or more instruments are 3D representations.

Anatomical structures and instruments employed during medical procedures inherently have a 3D shape. When these 3D shapes are projected onto a 2D plane, as happens during visualization on a traditional 2D display, a lot of information is lost. Often, it is necessary to present a 2D view from multiple angles in order to properly assess a situation. Even then, the geometric relations between the different objects shown on the 2D display may not be directly clear and unambiguous. The usage of a 3D display, in conjunction with 3D model and tracking data, solves these problems.

In some embodiments of the system employing a 3D display, the imaging data is 2D data that is processed to allow for visualization on the 3D display.

Typically, live imaging data obtained during a medical procedure is of a 2D nature. Displaying the 2D imaging data as 3D data could introduce visualization artifacts and/or give the false impression that additional geometric information is contained in the data. To visualize data on a 3D display, such as an autostereoscopic display or XR glasses or any other type of 3D visualization system, two virtual cameras are placed in the scene that is to be rendered. Each of these cameras will provide the image for a separate eye of the user. By making the positions and orientations of these cameras coincide, both eyes of the user are presented with the same images. The resulting image that is visualized on the display is thus perceived as a 2D image. This technique allows for the proper visualization of a 2D image on a 3D display. It also allows for the conversion of a native 3D image to a 2D image.

In some embodiments of the system, there is at least a partial overlap between two or more of the identified regions and the visualization comprises an overlay

3

of the visualization of the data originating from the overlapping regions. For instance, the amount of spatial overlap between the regions can be 0%, 20%, 50% or 100% of the surface area of the smallest of the regions. In case of overlap between three or more regions, the amount of overlap is not necessarily identical between every pair of regions among the three or more regions.

For instance, there may be a partial spatial overlap between the second region and the fourth region and the visualization comprises an overlay of the representation of the shape and/or the position and/or the orientation of at least one of the one or more instruments on a representation of the model data.

Preferably, there is a significant spatial overlap between the second region - comprising the inserted instruments - and the fourth region - comprising the model data. Preferably, there is also a significant spatial overlap between both regions and the target region of the medical procedure or the simulated medical procedure. Preferably, the overlay comprises a representation of the shape and position and orientation of all of the instruments inserted into the patient or the patient model, and a representation of the model data. The resulting visualization allows a physician to see in real time and 3D where and how the instruments are positioned within the model representation of the patient's body or the patient model. If the model data is acquired from actual measurements on the patient's body or the patient model and is sufficiently accurate, the resulting visualization allows a physician to interpret in real time and 3D where and how the instruments are positioned within the patient's body or the patient model.

For instance, there may be a partial spatial overlap between the first region and the second region and the visualization comprises an overlay of the representation of the shape and/or the position and/or the orientation of at least one of the one or more instruments on a representation of the imaging data.

Preferably, there is a significant spatial overlap between the second region - comprising the inserted instruments - and the first region - comprising the imaging data. Preferably, there is also a significant spatial overlap between both regions and the target region of the medical procedure or the simulated medical procedure. Preferably, the overlay comprises a representation of the shape and position and orientation of all of the instruments inserted into the patient or the patient model, and a representation of the imaging data. The resulting visualization allows a physician to see in real time where and how the instruments are positioned within the patient's body or the patient model.

In some embodiments of the system wherein the visualization comprises an overlay, the overlay is presented aligned on corresponding data or the overlay can be positioned and repositioned during the procedure such as to offer a desired view for a user.

Because of the co-registration of the different data streams, the transformations between the different data streams are known. It is thus possible to present the visualizations of two or more different data streams simultaneously where the visualization of data points that correspond to the same physical features are aligned. This allows for the direct and unambiguous verification of where and how the instruments are positioned within the patient's body or the patient model and of whether they risk to damage any of the surrounding tissues. Alternatively, both visualizations may be positioned independently of one another, without aligning the corresponding data points, in order to obtain a clearer view of the representation of one or more of the data streams.

The skilled person understands that it is possible to display more than two different data streams simultaneously in overlay. For instance, both the model data and the tracking data may be overlaid on the imaging data.

In some embodiments of the system, the latency of the system is equal to or smaller than 50 ms. Preferably, the latency of the system is inferior to 40 ms, more preferably inferior to 30 ms, most preferably inferior to 20 ms. The skilled person understands that the system latency may comprise the time needed for raw data generation and acquisition, eventual pre-processing of the raw data, the time required to estimate or calculate the transformations between the respective reference frames of the different data streams, the time required to co-register the different data streams, based on the obtained transformations and the time required for the rendering and visualization pipeline.

In order to be useful for providing real-time visual guidance during a medical procedure, it is imperative that the latency of the system is sufficiently small. Given the small size of some anatomical structures, a significant latency between the actions of the physician, and hence the receipt of the raw data, and the visualization of said data could lead to the physician advancing an instrument too far before feedback is received, thereby inducing an unintentional collision of the instrument with the tissue of the anatomical structure. By keeping the maximum total latency of the system in the same order of magnitude as the time the human brain needs to acquire and process an image, this risk is mitigated. In addition, perceptible system latency can be fatiguing and/or frustrating for the physician.

In some embodiments of the system, the co-registration of the model data, imaging data, tracking data and orientation data occurs in a continuous or intermittent manner during the procedure.

The time required for the estimation or calculation of the transformations between the respective reference frames of the different data streams makes a significant contribution to the total system latency. Therefore, the total system latency and/or the required computational power can be significantly reduced if the co-registration of the data streams is not updated for every frame that is displayed, but rather in an intermittent fashion. The intermittent co-registration process may be triggered in a variety of ways. It may for instance occur after a set number of frames have been visualized, or when prompted by a user of the system, or when a certain amount of movement of one of the reference frames has been detected.

In some embodiments of the system, the model data is generated prior to the procedure.

The generation of model data generally relies on a large amount of imaging data, wherein this imaging data is heavily processed. Therefore, the generation of model data is a computationally intensive task. The total system latency and/or the required computational power can thus be significantly reduced if model data is generated prior to the medical or simulated medical procedure.

In some embodiments of the system, the model data is generated using computed tomography, MRI or any other suitable modality.

In some embodiments of the system, the imaging data is generated during the surgical procedure.

In some embodiments of the system, the imaging data is generated using fluoroscopy or intraoperative MRI (iMRI) or any other real-time imaging method.

In some embodiments of the system, the imaging data is generated in an intermittent manner. In this context, generating imaging data "in an intermittent manner" is to be understood as opposed to continuously generating imaging data or generating imaging data per frame that is to be visualized. When operating in an intermittent manner, the generation of imaging data can for instance be controlled by an elapsed time interval, a displayed number of frames, a predetermined amount of movement of one of the instruments, a predetermined amount of variation between successively acquired data streams, a predetermined amount of variation between successive images or an action of the physician performing the procedure. The skilled person is capable of understanding the circumstances at play and of deriving a suitable criterion for the determination of the interval for the generation of imaging data.

There are multiple reasons to generate imaging data in an intermittent manner instead of continuously or on a per frame basis. First of all, typical imaging techniques such as fluoroscopy expose the patient to a non-negligible amount of radiation. By only generating imaging techniques in an intermittent manner, the total radiation exposure of the patient can be greatly reduced. Secondly, the preprocessing of the raw imaging data may be a computationally intensive task, which might negatively impact the total system latency. The total system latency and/or the required computational power can thus be significantly reduced if imaging data is generated in an intermittent fashion. In third place, typical imaging techniques require a finite exposure time. This exposure time may be longer than the total system latency that is desired.

In some embodiments of the system, one or more sensors for generating the tracking data are embedded in or fixedly attached to at least one of the one or more instruments. These sensors may comprise accelerometers, capacitive sensors, inductive sensors, optical sensors, LIDAR or any other kind of sensor that is capable of generating data that allows for the estimation of a position and/or orientation and/or shape.

This embedding or fixedly attaching of sensors ensures that the sensors do not separate from the instruments inside the patient's body. Furthermore, it ensures that the position of the sensors on the instrument is fixed, both in space and in time. Alternatively, the sensors may be attached to the instruments in a movable manner, provided the geometric relationship between the sensor and the instrument is known or can be determined. Consequently, periodic recalibration of the tracking points generated by the sensors with respect to the instrument's shape or orientation is not required.

Preferably, the sensors are embedded in the instruments. This permits the instruments to have a smooth outer surface which is not abrasive towards the tissues inside the patient's body. Furthermore, this ensures that the surface of the instrument does not need to display any substantial relief, gaps or crevices, which can become a breeding ground for pathogenic organisms.

In some embodiments of the system, at least one of the one or more sensors comprises an optical fiber and/or at least one of the one or more sensors comprises one or more electromagnetic trackers and/or at least one of the one or more sensors is a distributed sensor that conforms to the shape of the instrument.

Optical fibers are ideally suited for implementation in medical instruments. They are small in diameter, lightweight, flexible, chemically inert and do not emit electromagnetic noise. Furthermore, they offer a high bandwidth for data transfer and can be used to introduce a light source inside of the patient's body. When inscribed with multiple Fiber Bragg Gratings (FBG)s, distributed along the length of the fiber, the local strain on the fiber can be measured which allows for a reconstruction of the fiber's shape. Based on the estimated shape of the fiber, the position, orientation and shape of the instrument can be determined.

Another option is to use Electromagnetic Trackers (EMTs) embedded in the instrument. A thorough explanation of the combined usage of distributed FBG sensors and EMTs for shape reconstruction of a catheter can be found in:

Ha et al., Robust Catheter Tracking by Fusing Electromagnetic Tracking, Fiber Bragg Grating and Sparse Fluoroscopic Images, IEEE Sensors Journal, Vol. 21, Issue 20, Pages 23422-23434, DOI: 10.1109/JSEN.2021.3107036.

In some embodiments of the system, one or more cameras or LIDAR systems have an unobstructed view on at least one of the one or more instruments to derive the tracking data based on image processing techniques applied on the camera or LIDAR images.

In some procedures and/or for some instruments, it might be possible to position one or more cameras or LIDAR systems such that they retain an unobstructed view of the instrument, even when said instrument is inserted into the patient's body or the patient model. It is an advantage of this technique that it requires no additional sensors inside or on the instruments and can thus be employed with standard instruments that are in widespread use.

According to a second aspect of the invention, a method for providing a visualization during a medical procedure on a patient or simulated medical procedure on a patient model is disclosed, the method comprising the steps of:

a. receiving image data of a first region of the patient's body or the patient model;
b. receiving tracking data of one or more instruments that are inserted in a second region of the patient's body or the patient model;
c. receiving pose data of the patient's body or the patient model, belonging to a third region;
d. co-registering the imaging data, tracking data and pose data;
e. simultaneously visualizing of two or more of the first, second and third regions, and of the co-registered data belonging to the simultaneously visualized regions;
the method being characterized in that
f. the tracking data comprises three or more tracking points, , wherein the three tracking points are not colinear; and/or one or more tracking points and a roll, pitch and yaw angle or a set of Euler angles or a set of quaternions or an angle-axis representation or a rotation matrix or a homogeneous transformation matrix; and/or a point cloud representation, for at least one of the one or more instruments;
g. the tracking data is used to determine a position and/or shape and/or orientation of at least one of the one or more instruments; and
h. the visualization comprises a representation of the shape and/or the position and/or the orientation of at least one of the one or more instruments.

In some embodiments of the method, the method further comprises the steps of:

a. receiving model data of a fourth region of the patient's body or the patient model;
b. co-registering the imaging data, tracking data and pose data;
c. simultaneously visualizing two or more of the first, second, third and fourth regions, and of the co-registered data belonging to the simultaneously visualized regions.

**Brief description of the drawings**

Figure 1 schematically illustrates an embodiment of the system according to the present disclosure.

Figures 2(a) and 2(b) schematically illustrate a simulated medical procedure.

Figure 3(a) shows a patient model for use in simulated cardiac catheterization.

Figures 3(b)-(f) show various manners to visualize a simulated medical procedure on the patient model of Figure 3(a).

**Detailed description**

The present disclosure will be described in terms of specific embodiments, which are illustrative of the disclosure and which are not to be construed as limiting. It will be appreciated that the present disclosure is not limited by what has been particularly shown and/or described and that alternatives or modified embodiments could be developed in the light of the overall teaching of this disclosure. The drawings described are only schematic and are non-limiting.

Reference throughout this description to "one embodiment" or "an embodiment" means that a particular feature, structure or characteristic described in connection with the embodiments is included in one or more embodiment of the present disclosure. Thus, appearances of the phrases "in one embodiment" or "in an embodiment" in various places throughout this specification are not necessarily all referring to the same embodiment, but may do so. Furthermore, the particular features, structures or characteristics may be combined in any suitable manner, as would be apparent to one of ordinary skill in the art from this disclosure, in one or more embodiments.

Specific features, structures or characteristics are indicated in the figures by reference numerals. In order not to overload the figures, not every feature is indicated in every figure. Conversely, in order not to overload the text, not every feature indicated in a figure is also discussed in the context of this particular figure.

Finally, the use of ordinal numbers such as "first", "second" and the like throughout this disclosure in no way implies a hierarchical relationship - not in terms of importance, position or time - between the features with which they are used, unless explicitly stated to the contrary. These ordinal numbers serve only to differentiate between different but similar features, properties, or structures.

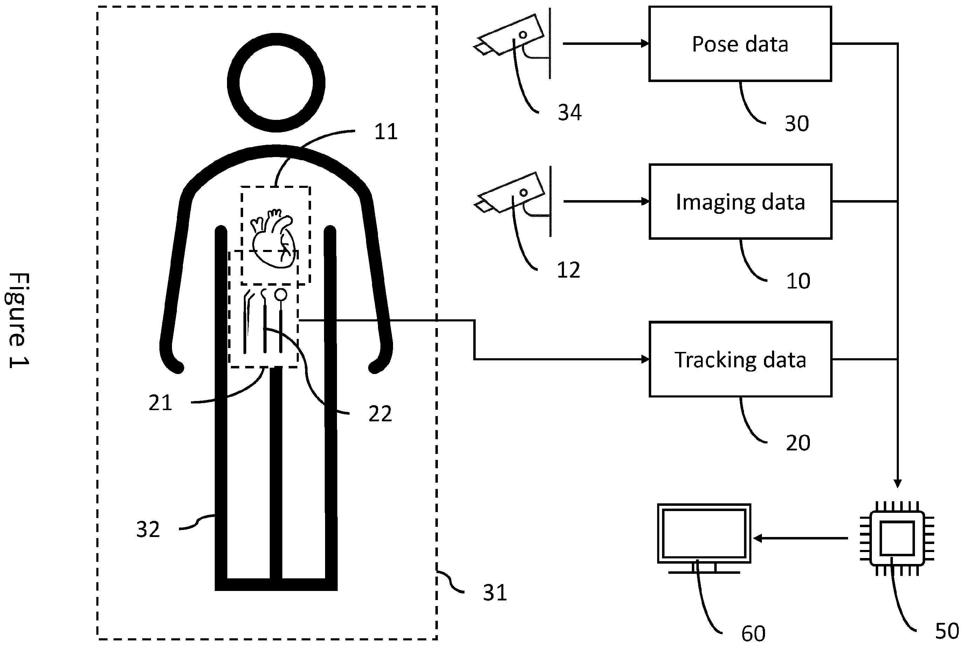

Figure 1 schematically illustrates an embodiment of the system according to the present disclosure. In the embodiment of Figure 1, a patient undergoes a medical procedure.

A first imager 12 provides imaging data 10 of a first region 11 of the patient's body 32. The first imager 12 can be for instance a fluoroscope or an intraoperative magnetic resonance imaging (iMRI) apparatus. Preferably, the first region 11 comprises the target region of the medical procedure.

A second imager 34 provides pose data 30 of the patient's body 32, belonging to a third region 31. The second imager can be for instance a camera or, more preferably, two camera's that are arranged to obtain a stereoscopic view of the patient's body 32. Preferably, the pose data 30 comprises at least data on the position and orientation of the body part that comprises the target region of the medical procedure. Preferably, the pose data 30 comprises data on the position and orientation of the patient's trunk and head.

One or more instruments 22 are inserted in a second region 21 of the patient's body 32. The instruments 22 can comprise any kind of surgical or non-surgical tool, such as for instance catheters, cannulas, needles, endoscopes, cutters, graspers, clamps, dilators and the like. Preferably, the second region coincides at least partially with the first region 11. Preferably, the second region 22 comprises the target region of the medical procedure.

At least one of the instruments 22 is equipped with means to generate tracking data 20. The tracking data 20 of a single instrument comprises the positions of at least three non-colinear tracking points. The knowledge of the position of three non-colinear points allows the unambiguous reconstruction of the position and orientation of the instrument. In addition, the three non-linear points may allow for the shape of the instrument to be reconstructed. In case the instrument is flexible or comprises articulations, more tracking points may be required to reconstruct its position, orientation or shape. Alternatively, when the shape of the instrument is known, the tracking data 20 of a single instrument may comprise the position of one or more tracking points and the values of a roll, a pitch and a yaw angle. Again, in case the instrument is flexible or comprises articulations, more tracking points and more angles may be required to reconstruct its position, orientation or shape. Specific techniques for generating tracking data will be discussed further in this disclosure.

The imaging data 10, tracking data 20 and pose data 30 are received by a processor 50. Each of these data streams belongs to a reference frame, which is related to the position, orientation and properties of the device that generated the data stream. Important differences may exist between the different reference frames; for instance the imaging data 10 and tracking data 20 may be translated or rotated with respect to one another or may differ in scale. In addition, continuously or intermittently acquired data streams may be sampled at different frequencies. The processor 50 co-registers the three different data streams to a single reference frame. This implies that, where necessary, the imaging data 10, tracking data 20 and pose data 30 are rotated, translated and scaled - possibly both in the spatial and the temporal domain - to properly align the three data streams. The co-registration process will be illustrated in more detail further in this disclosure.

Finally, two or more of the co-registered data streams are simultaneously visualized on the display 60. Specific examples of simultaneously visualized data are given further in this disclosure.

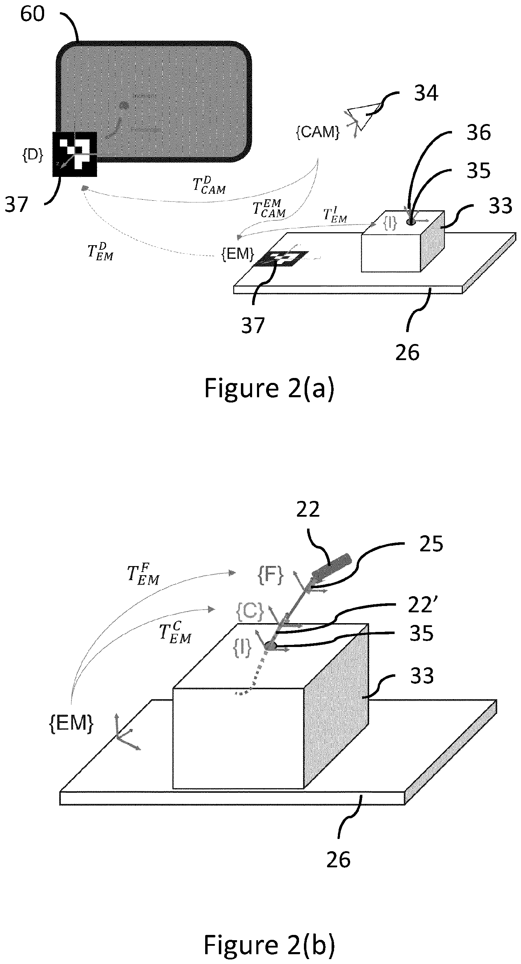

Figures 2(a) and 2(b) schematically illustrates an example of a simulated medical procedure. In this simulated medical procedure, a virtual fetoscope 22 is inserted into a patient model 33, which takes the form of a simple cubic cavity in the embodiment of Figures 2(a) and 2(b). The fetoscope 22 is inserted into the patient model 33 through a cannula 22' at the incision point 35. The fetoscope 22 and the incision point 35 are provided with respective electromagnetic (EM) trackers 25 and 36. The patient model 33 is placed on an EM field generator 26, which serves to track the position of both EM trackers 25 and 36.

In this embodiment, multiple reference frames exist: the incision point reference frame {I}, the cannula reference frame {C}, the fetoscope reference frame {F}, the EM field generator reference frame {EM} and the display reference frame {D}. In order to provide proper visual guidance, data originating in different reference frames needs to be co-registered. This requires knowledge of the transformation matrices between the different reference frames.

In order to calculate the transformation matrix $T_{EM}^{D}$ between the EM field generator 26 and the display 60, both the display and the generator are equipped with a tracker 37. A camera 34 is positioned such that it can observe both markers; the camera itself has a reference frame {CAM}. Since one of the markers 37 is fixedly attached to the EM field generator 26, the transformation matrix $T_{EM}^{M}$ between the generator and that marker is known. By tracking both markers 37 with the camera 34, the transformation matrices $T_{CAM}^{D}$ and $T_{CAM}^{EM}$, which are the transforms from {CAM} to {D} and {EM} respectively, can be calculated. Then the transformation matrix between {EM} and {D} can be obtained as $T_{EM}^{D} = (T_{CAM}^{EM})^{-1} T_{CAM}^{D}$. Knowledge of this transformation matrix allows for the coregistration of data which originates from the {EM} reference frame, in this case the pose data of the patient model 33 - which is fixedly attached to the generator, in the reference frame of the display 60. By continuous tracking of the markers 37, the transformation matrix is always known and calibration of the system is possible whenever the display 60 or the EM generator 26 are moved.

In the setup as shown, it is most convenient to first transform data originating from the reference frames of the incision point, the cannula or the fetoscope, to the reference frame of the EM generator. The incision point EM tracker 36 provides the transformation matrix $T_{EM}^I$ and the fetoscope EM tracker 25 provides the transformation matrix $T_{EM}^F$. The position and orientation of the cannula 22' and the fetoscope 22 are visualized with respect to the incision point 35. When visualizing the 3D augmented reality (AR) content on the display 60, the incision point remains static and only the rotation and insertion of the cannula 22' and the fetoscope 22 are represented. The rotations are preferably calculated in {D} to ensure that the AR instruments are always parallel with the real instruments. Since the fetoscope 22 is inserted through the cannula 22', the two instruments are collinear, thus their rotations are the same. Therefore, the transformation matrix from the reference frames of the fetoscope and the cannula to the reference frame of the display can be expressed as $T_D^F = T_D^{EM} T_{EM}^F$. The insertion length of the instruments is calculated as the distance between the calibrated incision point 35 and the points defined by the fetoscope EM sensor 25. These translations are expressed in {EM}. The obtained distances are the length of the instruments that are outside of the patient, $l_{F,o}$ and $l_{F,o}$, for the fetoscope 22 and cannula 22', respectively. By knowing the total length of these instruments, the insertion lengths are calculated as $l_{F,i} = l_F - l_{F,o}$ and $l_{C,i} = l_C - l_{C,o}$, where $l_F$ and $l_C$ are the total length of the fetoscope and the cannula, respectively.

The fetoscope 22 consists of two parts, namely the rigid shaft and the flexible tip. The flexible tip consists of 10 cylinders, where each of the cylinders can rotate around the local z axis. This property is used to visualize the bending of the fetoscope, which has a range of 90 degrees. This bending angle is determined through measurement of the physician's input. The virtual fetoscope 22 is assumed to behave according to a constant curvature model, where the rotation angle of each of the cylinders is equal and where the flexible tip can only bend in one plane. Under this assumption, the bending angle is then divided by the number of cylinders, and given as a rotation to each cylinder. Since each consecutive cylinder is a child of the previous one, the resulting global bending angle of the 10th cylinder will be the same as the bending angle of the fetoscope: $\theta_s = \theta_F/n_s$, where $\theta_s$ and $\theta_F$ are the rotation angle of each segment and the overall bending angle of the flexible tip, respectively, while $n_s$ is the number of segments that the flexible tip is composed of. The orientation of the plane in which the virtual fetoscope 22 can bend is given by the fetoscope EM sensor 25.

The obtained insertion length, orientation and bending angle (from which the shape is known) of the fetoscope 22, making up the tracking data, are transformed to the display reference frame. The real time 2D fetoscopic view - the image obtained by the tip of the fetoscope 22 - is the imaging data in this embodiment. This imaging data is also transformed to the display reference frame and is set as the background of the visualization of the display. To generate the 3D view, two virtual cameras are placed in the scene with a horizontal offset of 65 mm, which corresponds to the average interpupillary distance. The offset can be adjusted based on user preference. The two views from the cameras are stacked side-by-side generating the 3D image input for an autostereoscopic display. If the 2D fetoscopy image is to be visualized on a 3D display, such as for instance an autostereoscopic display or VR glasses, two additional virtual cameras are placed in the renderer. The image of one of these cameras will be visualized for the left eye of the user, the image of the other camera will be visualized for the right eye of the user. By setting both cameras to an identical position and orientation, an identical view is provided for the left eye and the right eye which results in the perception of a 2D image, even when using a 3D display. Alternatively, only one additional virtual camera can be placed in the scene and the image of this camera can be duplicated for the left and right eye of the user. Alternatively, the raw fetoscopy image may be visualized for both the left and right eye of the user. Furthermore, the visualization of the instrument can be positioned outside of the visualization of the imaging data or on top of the visualization of the imaging data, as the user can freely move it in the scene. This hybrid 2D and 3D visualization is preferably presented on an autostereoscopic display.

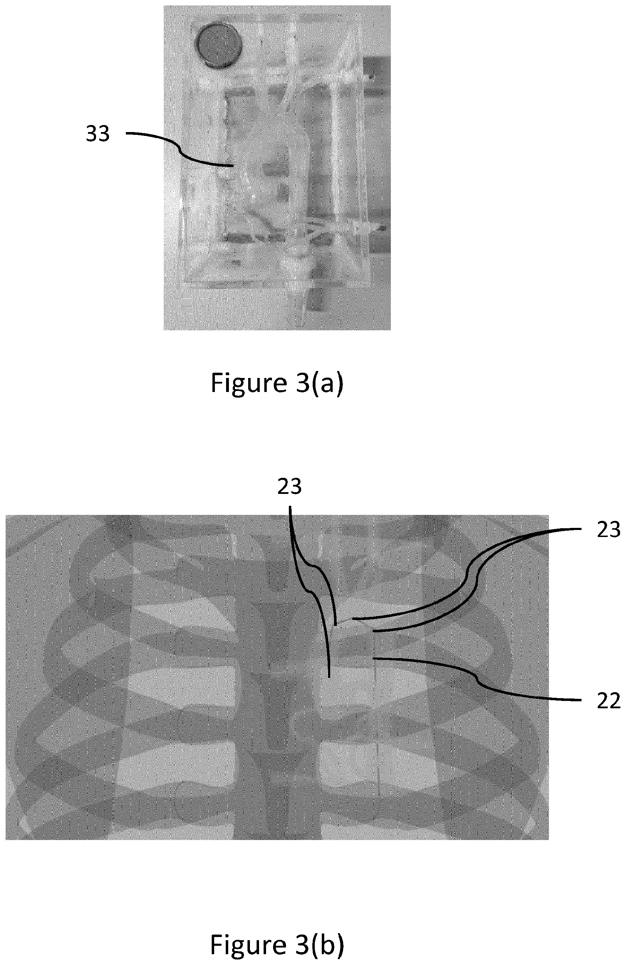

Figure 3(a) shows a picture of a patient model 33. In the embodiment of Figure 3(a), the patient model is a box trainer with a silicon coronary vessel model, employed for the simulation of cardiac catheterization. Figures 3(b)-3(f) all refer to the same embodiment.

A catheter 22 is inserted into the silicon vessel model. The catheter 22 comprises a distributed Fiber Bragg Grating (FBG) sensor that returns tracking data comprising the 3D positions of tracking points 23. The tracking data comprises at least three or more tracking points. Preferably, the spacing of the tracking points along the length of the catheter is adapted to the mechanical properties of the catheter such that every bending mode of the catheter can be tracked and visualized. For instance, the tracking data may comprise tracking points that are spaced one millimeter apart along the length direction of the catheter. Preferably, the tracking points are not located along the centerline of the catheter but are distributed at a lateral distance of the centerline along two or more sides of the centerline. Such a distribution of tracking points ensures that three non-colinear tracking points are available at any time - also when the catheter is not bent. However, even when only a single fiber is located along the centerline of the catheter, the fiber will return non-colinear points once it is bent and colinear points when it is straight, making this arrangement useful as well for determining the position, orientation and shape of the catheter.

Typically, intra-operative imaging data for a cardiac catheterization is delivered by a C-arm fluoroscope, which delivers 2D fluoroscopy images. For the purpose of a simulated catheterization, the fluoroscopy image can be simulated using the patient model 33 and a model of a ribcage. In Figure 3(b), such a simulated fluoroscopy image is visualized. The imaging data and the tracking data of the catheter are co-registered and a visualization of the catheter 22 is added, based on the obtained 3D positions of the tracking points 23. The visualization of the catheter displays the position, orientation and shape of the catheter within the coronary vessels. Preferably, the view can be rotated along the x, y and z axes, thereby simulating the movements of a C-arm fluoroscope.

If the 2D fluoroscopy image is to be visualized on a 3D display, such as for instance an autostereoscopic display or VR glasses, two virtual cameras are placed in the renderer. The image of one of these cameras will be visualized for the left eye of the user, the image of the other camera will be visualized for the right eye of the user. By setting both cameras to an identical position and orientation, an identical view is provided for the left eye and the right eye which results in the perception of a 2D image, even when using a 3D display. In this case the visualization of the catheter is also converted to a 3D visualization.

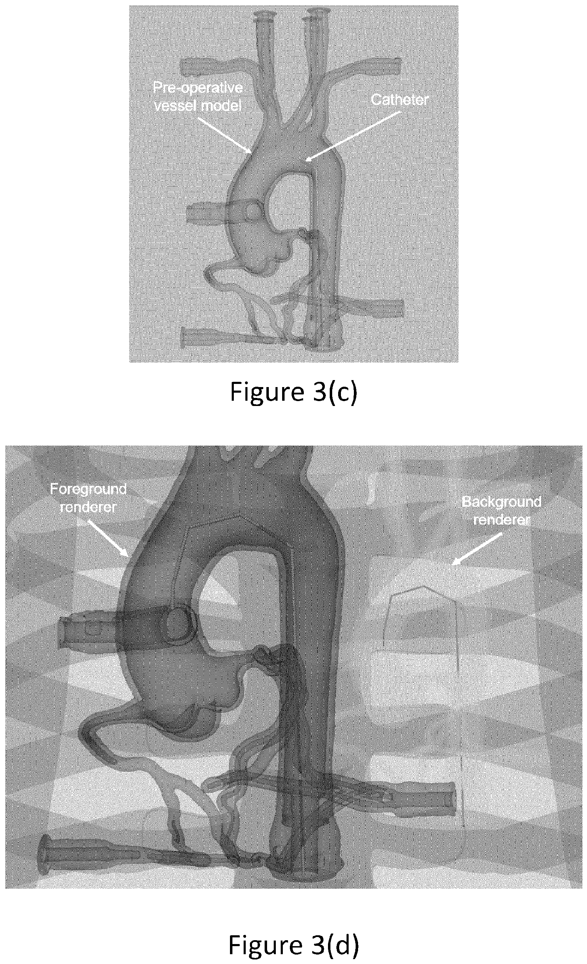

Through pre-operative imaging, model data of the coronary vessels can be obtained, for instance by means of CT 3D reconstruction. Figure 3(c) shows a visualization of a 3D model of the patient model. Preferably, the color, transparency, orientation and scale of the visualization can be changed based on the preference of the user. The tracking data of the catheter is co-registered with the model data and visualized in the same scene. If the 3D model data and 3D tracking data are to be visualized on a 3D display, two virtual cameras are placed in the renderer. The image of one of these cameras will be visualized for the left eye of the user, the image of the other camera will be visualized for the right eye of the user. A translation is introduced between the positions of both cameras. Preferably, the translation can be adjusted based on the interpupillary distance (IPD) of the user. Preferably, the default translation between the positions of both virtual cameras is 65mm, which corresponds to the mean IPD of humans. Since the contents of the left and right image are shifted with respect to one another, the user perceives a 3D image.

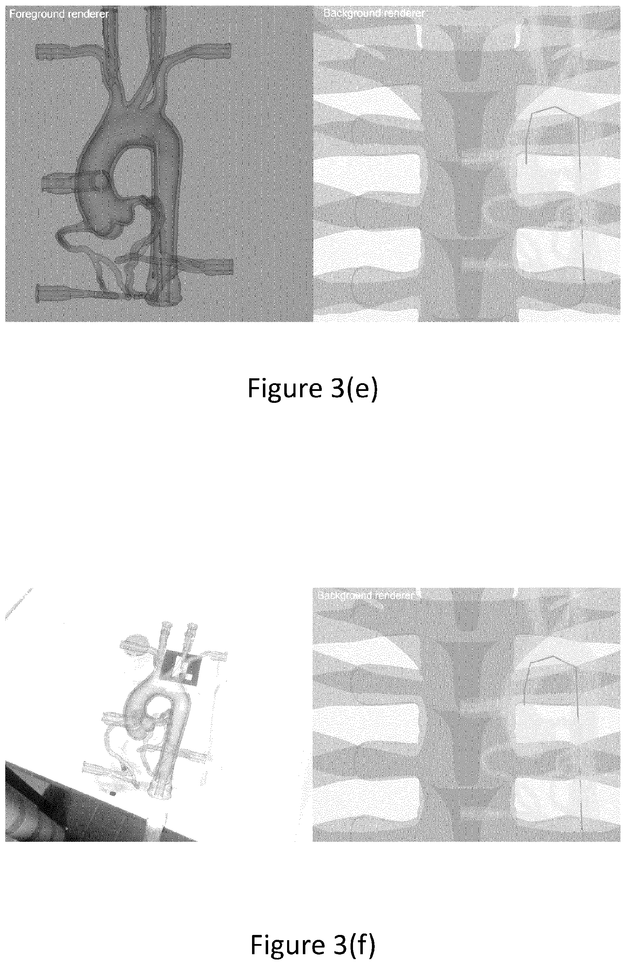

It is also possible to combine the two formerly mentioned visualization options in a single visualization. In Figure 3(d), the background renderer comprises a 2D visualization of the 2D imaging data, co-registered with the 3D tracking data. The foreground renderer comprises a 3D visualization of the 3D model data, co-registered with the 3D tracking data. Alternatively, both visualizations can be displayed side-by-side, as shown in Figure 3(e). This visualization mode is useful to avoid possible occlusions. Also in this visualization mode, the 3D model data can be rotated and scaled in accordance with user preferences or to align the 3D model data with the imaging data. Yet another visualization possibility is illustrated in Figure 3(f). On the left, the foreground renderer comprises a visualization of the pose data of the patient model, which can be captured using a 2D or 3D camera. The tracking data is co-registered with the pose data and visualized on the same image. On the right, the background renderer comprises a 2D visualization of the 2D imaging data, co-registered with the tracking data. Preferably, the user can freely switch between the illustrated viewing modes.

Reference signs

| | |
|---|---|
| imaging data | 10 |
| first region | 11 |
| imager | 12 |
| tracking data | 20 |
| second region | 21 |
| instruments | 22 |
| tracking points | 23 |
| instrument electromagnetic tracker | 25 |
| electromagnetic field generator | 26 |
| pose data | 30 |
| third region | 31 |
| patient's body | 32 |
| patient model | 33 |
| camera | 34 |
| incision point | 35 |
| incision point electromagnetic tracker | 36 |
| tracker | 37 |
| processor | 50 |
| display | 60 |

**Claims**

1. A system for providing a visualization during a medical procedure on a patient or a simulated medical procedure on a patient model, the system comprising:

    a. means for receiving imaging data of a first region of the patient's body or the patient model;
    b. means for receiving tracking data of one or more instruments that are inserted in a second region of the patient's body or the patient model;
    c. means for receiving pose data of the patient's body or the patient model, the pose data belonging to a third region;
    d. processing means for co-registering the imaging data, tracking data and pose data;
    e. a display for simultaneous visualization of two or more of the first, second and third regions, and of the co-registered data belonging to the simultaneously visualized regions;

**characterized in that**

f. the tracking data comprises three or more tracking points, wherein the three tracking points are not colinear; and/or one or more tracking points and a roll, pitch and yaw angle or a set of Euler angles or a set of quaternions or an angle-axis representation or a rotation matrix or a homogeneous transformation matrix; and/or a point cloud representation, for at least one of the one or more instruments;

g. the tracking data is used to determine a position and/or shape and/or orientation of at least one of the one or more instruments; and

h. the visualization comprises a representation of the shape and/or the position and/or the orientation of at least one of the one or more instruments.

2. The system according to claim 1, the system further comprising means for receiving model data of a fourth region of the patient's body or the patient model, wherein the processing means are adapted for co-registering the imaging data, tracking data, pose data and model data, wherein the display is adapted for simultaneous visualization of two or more of the first, second, third and fourth regions, and of the co-registered data belonging to the simultaneously visualized regions.

3. The system according to claim 2,

a. wherein the model data and tracking data comprise 3D data;

b. wherein the display is a 3D display; and

c. wherein a representation of the model data and the representation of the shape and/or position and/or orientation of the one or more instruments are 3D representations.

4. The system according to claim 3, wherein the imaging data is 2D data that is that is processed to allow for visualization on the 3D display.

5. The system according to any of the previous claims, wherein there is at least a partial overlap between two or more regions and wherein the visualization comprises an overlay of the representation of the data originating from the two or more regions.

6. The system according to claim 5, wherein the overlay is presented aligned on corresponding data or wherein the overlay can be positioned and repositioned during the procedure such as to offer a desired view for a user.

7. The system according to any of the previous claims, wherein the system latency is equal to or smaller than 50 ms.

8. The system according to any of the previous claims, wherein the co-registration of the model data, imaging data, tracking data and orientation data occurs in a continuous or intermittent manner during the procedure.

9. The system according to any of the previous claims, wherein the model data is generated prior to the procedure.

10. The system according to any of the previous claims, wherein the imaging data is generated during the surgical procedure.

11. The system according to claim 10, wherein the imaging data is generated in an intermittent manner.

12. The system according to any of the previous claims, wherein one or more sensors for generating the tracking data are embedded in or fixedly attached to at least one of the one or more instruments.

13. The system according to claim 12, wherein at least one of the one or more sensors comprises an optical fiber and/or wherein at least one of the one or more sensors comprises one or more electromagnetic trackers and/or wherein at least one of the one or more sensors is a distributed sensor that conforms to the shape of the instrument.

14. The system according to any of the previous claims, wherein one or more cameras or LIDAR systems have an unobstructed view on at least one of the one or more instruments to derive the tracking data based on image processing techniques applied on the camera or LIDAR images.

15. A method for providing a visualization during a medical procedure on a patient or simulated medical procedure on a patient model, the method comprising the steps of:

a. receiving image data of a first region of the patient's body or the patient model;

b. receiving tracking data of one or more instruments that are inserted in a second region of the patient's body or the patient model;

c. receiving pose data of the patient's body or the patient model, belonging to a third region;

d. co-registering the imaging data, tracking data and pose data;

e. simultaneously visualizing of two or more of the first, second and third regions, and of the co-registered data belonging to the simultaneously visualized regions;

the method being **characterized in that**

f. the tracking data comprises three or more tracking points, wherein the three tracking points are not colinear; and/or one or more tracking points and a roll, pitch and yaw angle or a set of Euler angles or a set of quaternions or an angle-axis representation or a rotation matrix or a homogeneous transformation matrix; and/or a point cloud representation, for at least one of the one or more instruments;

g. the tracking data is used to determine a position and/or shape and/or orientation of at least one of the one or more instruments; and

h. the visualization comprises a representation of the shape and/or the position and/or the orientation of at least one of the one or more instruments.

Figure 1

Figure 2(a)

Figure 2(b)

Figure 3(a)

Figure 3(b)

Figure 3(c)

Figure 3(d)

Figure 3(e)

Figure 3(f)

Europäisches
Patentamt

European
Patent Office

Office européen
des brevets

# EUROPEAN SEARCH REPORT

**Application Number**

EP 23 15 3173

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/192226 A1 (LANG PHILIPP K [US]) 27 June 2019 (2019-06-27) * abstract; figures 11, 14 * * paragraphs [0219], [0220], [0912], [0969], [1183], [1184], [1293] * | 1-12,14, 15 | INV. A61B34/20 A61B90/00 G09B23/28 |
| X | US 2020/188028 A1 (FEINER STEVEN [US] ET AL) 18 June 2020 (2020-06-18) * abstract; figure 3 * * paragraphs [0035], [0036], [0039], [0041], [0051] * | 1-15 | |
| A | HA XUAN THAO ET AL: "Robust Catheter Tracking by Fusing Electromagnetic Tracking, Fiber Bragg Grating and Sparse Fluoroscopic Images", IEEE SENSORS JOURNAL, IEEE, USA, vol. 21, no. 20, 24 August 2021 (2021-08-24), pages 23422-23434, XP011884243, ISSN: 1530-437X, DOI: 10.1109/JSEN.2021.3107036 [retrieved on 2021-10-18] * the whole document * | 1-15 | |

**TECHNICAL FIELDS SEARCHED (IPC)**

A61B

The present search report has been drawn up for all claims

| Place of search | Date of completion of the search | Examiner |
|---|---|---|
| Munich | 3 July 2023 | Hilbig, Sophie |

EPO FORM 1503 03.82 (P04C01)

## ANNEX TO THE EUROPEAN SEARCH REPORT
## ON EUROPEAN PATENT APPLICATION NO.

EP 23 15 3173

This annex lists the patent family members relating to the patent documents cited in the above-mentioned European search report.
The members are as contained in the European Patent Office EDP file on
The European Patent Office is in no way liable for these particulars which are merely given for the purpose of information.

03-07-2023

| Patent document cited in search report | | Publication date | Patent family member(s) | | Publication date |
|---|---|---|---|---|---|
| US 2019192226 | A1 | 27-06-2019 | CA | 3016604 A1 | 21-09-2017 |
| | | | CN | 109310476 A | 05-02-2019 |
| | | | CN | 111329551 A | 26-06-2020 |
| | | | CN | 111329552 A | 26-06-2020 |
| | | | CN | 111329553 A | 26-06-2020 |
| | | | CN | 111329554 A | 26-06-2020 |
| | | | EP | 3426179 A1 | 16-01-2019 |
| | | | US | 2017258526 A1 | 14-09-2017 |
| | | | US | 2018116728 A1 | 03-05-2018 |
| | | | US | 2018125584 A1 | 10-05-2018 |
| | | | US | 2018263704 A1 | 20-09-2018 |
| | | | US | 2019110842 A1 | 18-04-2019 |
| | | | US | 2019192226 A1 | 27-06-2019 |
| | | | US | 2019262078 A1 | 29-08-2019 |
| | | | US | 2019380784 A1 | 19-12-2019 |
| | | | US | 2020060767 A1 | 27-02-2020 |
| | | | US | 2020246074 A1 | 06-08-2020 |
| | | | US | 2020305980 A1 | 01-10-2020 |
| | | | US | 2021022808 A1 | 28-01-2021 |
| | | | US | 2021106386 A1 | 15-04-2021 |
| | | | US | 2021267691 A1 | 02-09-2021 |
| | | | US | 2022087746 A1 | 24-03-2022 |
| | | | US | 2022249171 A1 | 11-08-2022 |
| | | | US | 2023038678 A1 | 09-02-2023 |
| | | | WO | 2017160651 A1 | 21-09-2017 |
| US 2020188028 | A1 | 18-06-2020 | US | 2020188028 A1 | 18-06-2020 |
| | | | WO | 2019040493 A1 | 28-02-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 20200188028 A1

**Non-patent literature cited in the description**

• **HA et al.** Robust Catheter Tracking by Fusing Electromagnetic Tracking, Fiber Bragg Grating and Sparse Fluoroscopic Images. *IEEE Sensors Journal,* vol. 21 (20), 23422-23434